# EUROPÄISCHE PATENTSCHRIFT

(11) **EP 1 246 578 B1**
(45) Veröffentlichungstag und Bekanntmachung des Hinweises auf die Patenterteilung: **10.09.2003**
(21) Anmeldenummer: 01993427.2
(22) Anmeldetag: 12.11.2001
(51) Int. Cl.: A61B 17/86

(54) **KNOCHENSCHRAUBE**
BONE SCREW
VIS POUR OS

(30) Priorität: 10.11.2000 DE 10055891
(43) Veröffentlichungstag der Anmeldung: 09.10.2002
(73) Patentinhaber: BIEDERMANN MOTECH GmbH, 78054 Villingen-Schwenningen (DE)
(72) Erfinder: BIEDERMANN, Lutz, 78048 VS-Villingen (DE); HARMS, Jürgen, 76227 Karlsruhe (DE); LINDSEY, Ronald, W., Houston, TX 77030 (US)
(74) Vertreter: Prüfer, Lutz H., Dipl.-Phys.
(86) Internationale Anmeldenummer: EP0113080
(87) Internationale Veröffentlichungsnummer: WO02038054

(56) Entgegenhaltungen:
- WO-A-00/10473
- WO-A-02/11630
- US-A- 5 968 047
- US-A- 6 086 589
- US-A- 6 120 502

## Beschreibung

Die Erfindung betrifft eine Knochenschraube mit einem Gewindeabschnitt, einer Spitze an einem ersten Ende und einem Kopf zum Eingreifen mit einem Schraubendreher am gegenüberliegenden zweiten Ende.

Aus der EP-A-0 682 917 (Basis für den Obergriff der Ansprüche 1 und 4) ist ein hohlzylindrischer Bolzen bekannt, der ein in einem Abstand von seiner Spitze beginnendes äußeres Knochengewinde sowie Ausnehmungen in seinem Mantel zum Ermöglichen der Knochenintegration aufweist. Die Vorrichtung ist so ausgebildet, daß ein Einschrauben nur in ein vorher zu fertigendes Bohrloch möglich ist.

Aus der WO 97/37603 ist eine Knochenschraube bekannt, deren Schaft eine durch Bohren hergestellte Mehrzahl von Perforationen zum Einwachsen aufweist.

Aus der US-6,048,343 ist ein Knochenschraubensystem mit einer kannelierten Knochenschraube bekannt.

Aufgabe der Erfindung ist es, eine Knochenschraube der eingangs beschriebenen Art zu verbessern.

Diese Aufgabe wird durch eine Knochenschraube gelöst, bei der der rohrförmige Gewindeabschnitt auf seiner Außenwandung ein Knochengewinde und wenigstens an einem Ende einen Innengewindeabschnitt zum Einschrauben von Kopf bzw. Spitze aufweist. Dadurch wird eine große Variabilität erreicht, da verschiedene Köpfe bzw. Spitzen einschraubbar sind.

Nach einer Weiterbildung erstreckt sich das Innengewinde über die gesamte Länge des Gewindeabschnittes. Dadurch wird der Vorteil erreicht, daß der Gewindeabschnitt auf jede gewünschte Länge vor Ort kürzbar ist, so daß auch hier die Variabilität erhöht wird.

Nach einer anderen Lösung ist die Knochenschraube dadurch gekennzeichnet, daß der rohrförmige Gewindeabschnitt auf seiner Außenwandung ein Knochengewinde aufweist und die Spitze als selbstschneidende Spitze ausgebildet ist. Dadurch entfällt die Notwendigkeit, zunächst eine Bohrung zur Aufnahme der Schraube vorzunehmen. Eine weitere Lösung ist dadurch gekennzeichnet, daß der rohrförmige Gewindeabschnitt auf seiner Außenwandung ein Knochengewinde aufweist und der Kopf und/oder die Spitze so bemessen sind, daß sie im Paßsitz in die Enden des Gewindeabschnittes einsetzbar sind. Hier wird wiederum die Variabilität der Einsetzbarkeit erhöht, indem der Gewindeabschnitt vor Ort auf eine gewünschte Länge kürzbar ist und dann Kopf bzw. Spitze der gewünschten Art einsetzbar sind.

Weiterbildungen der Erfindung sind in den Unteransprüchen gekennzeichnet.

Weitere Merkmale und Zweckmäßigkeiten der Erfindung ergeben sich aus der Beschreibung von Ausführungsbeispielen anhand der Figuren.

Von den Figuren zeigen:
- Fig. 1: eine Explosionsdarstellung;
- Fig. 2: eine zusammengesetzte Schraube einer ersten Ausführungsform;
- Fig. 3: eine zusammengesetzte Schraube einer zweiten Ausführungsform;
- Fig. 4: ein Detail aus Fig. 1 in vergrößertem Maßstab; und
- Fig. 5: ein Detail aus Fig. 1 im Schnitt in vergrößertem Maßstab.

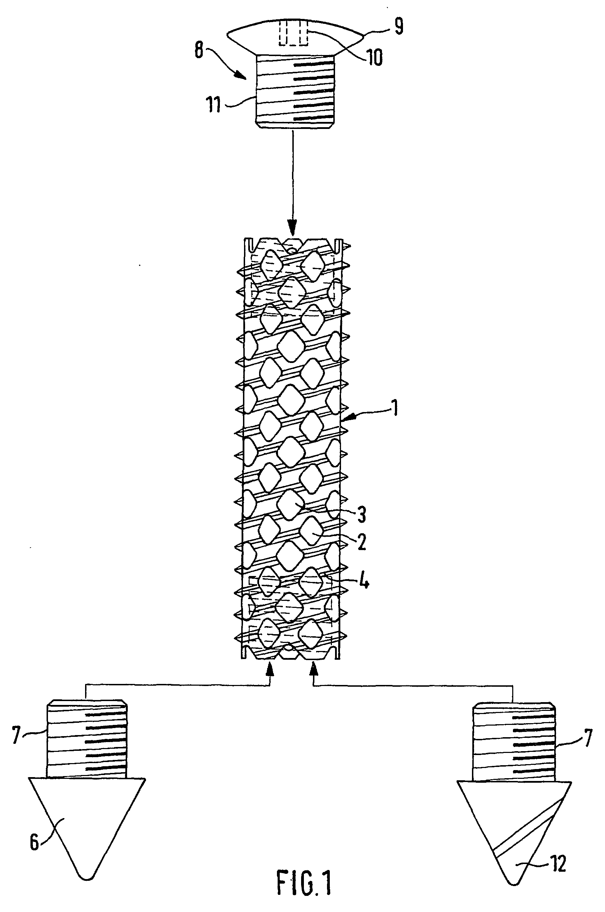

Wie am besten aus Fig. 1 ersichtlich ist, weist die Knochenschraube einen rohrförmigen Gewindeabschnitt 1 auf. Dieser weist in seiner Wandung eine Vielzahl von Ausnehmungen 2 auf, die in dem gezeigten Ausführungsbeispiel rautenförmig ausgebildet sind. Dabei erfolgt die Ausrichtung der Rauten derart, daß jeweils eine Symmetrieachse sich parallel zur Symmetrieachse des Rohres erstreckt. Ein erster Satz von Ausnehmungen 2 ist in Umfangsrichtung gegeneinander versetzt. In axialer Richtung gesehen angrenzend folgt ein weiterer Satz von Ausnehmungen 3, die in Umfangsrichtung gesehen den gleichen Abstand aufweisen, die aber so angeordnet sind, daß jeweils in axialer Richtung gesehen eine Öffnung des zweiten Satzes 3 zwischen zwei Öffnungen des vorhergehenden Satzes 2 liegen. Das Muster setzt sich über die ganze Fläche fort. Obwohl die Öffnungen vorzugsweise rautenförmig ausgebildet sind, können nach anderen Ausführungsbeispielen auch andere Öffnungsformen, insbesondere runde Öffnungen, vorgesehen sein.

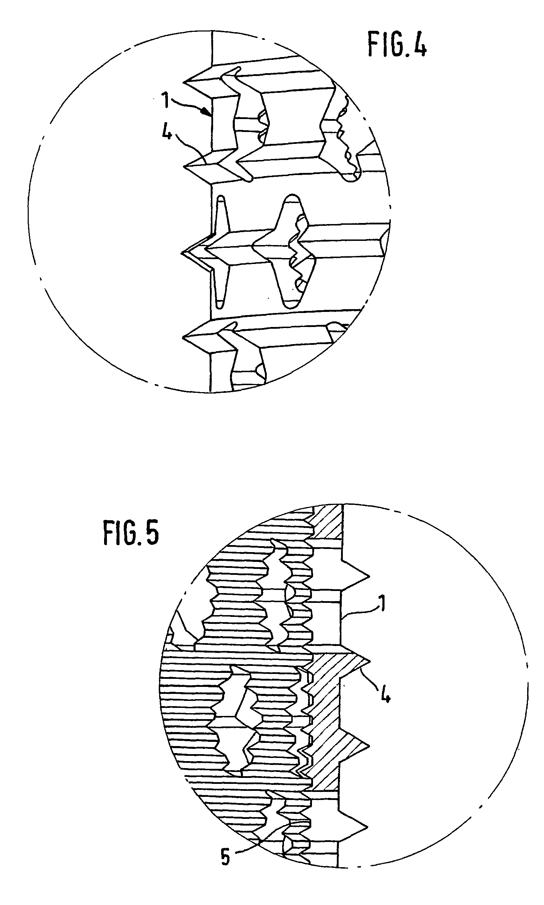

Auf der Außenwandung ist ein sogenanntes Knochengewinde 4 vorgesehen, welches in seiner Form den üblichen Knochenschrauben entspricht. Das Knochengewinde ist im Detail in Fig. 4 gezeigt.

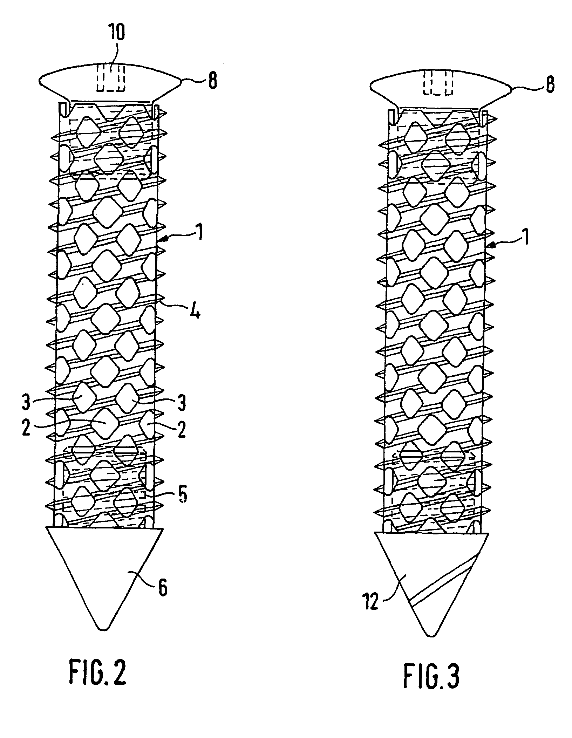

Die Knochenschraube umfaßt ferner eine an einem Ende des Rohres eingebrachte Spitze 6. Die Spitze umfaßt den eigentlichen Spitzenteil und einen Schaft 7. In dem gezeigten Ausführungsbeispiel weist der Schaft 7 ein metrisches Außengewinde auf. Der rohrförmige Gewindeabschnitt 1 weist auf seiner Innenwandung ein entsprechendes metrisches Innengewinde auf, und die Spitze und das Rohr sind in der in Fig. 2 ersichtlichen Weise durch Einschrauben der Spitze miteinander fest verbunden. Dabei sind die beiden metrischen Gewinde entgegen der Rechtsgewindesteigung des Knochengewindes 4 als Linksgewinde ausgebildet.

Die Knochenschraube umfaßt ferner einen Kopf 8, der, wie am besten aus Fig. 1 ersichtlich ist, den eigentlichen Kopfteil 9 mit einem Schlitz oder einem Innen-Sechskant und einen Gewindeschaft 11 aufweist. Das den Gewindeabschnitt 1 bildende Rohr weist an seinem zugehörigen Ende ein zu dem Gewinde des Gewindeschaftes 11 passendes metrisches Innengewinde auf. Auch die beiden zusammenwirkenden Gewinde des Gewindeschaftes 11 und das Innengewinde sind vorzugsweise als Linksgewinde ausgebildet. Der Kopf 8 ist in der in Fig. 2 gezeigten Weise in den Gewindeabschnitt 1 fest eingeschraubt.

Die in Fig. 3 gezeigte Ausführungsform unterscheidet sich von der zuerst beschriebenen Ausführungsform dadurch, daß die Spitze 12 als eine selbstschneidende Spitze ausgebildet ist. Bei dieser Ausführungsform und auch bei der ersten Ausführungsform sind die Spitzen jeweils in der in den Figuren gezeigten Weise abgerundet.

Bei den oben beschriebenen Ausführungsformen sind die Spitze und der Kopf eingeschraubt. Das metrische Innengewinde erstreckt sich jeweils so über die beiden Enden, daß Kopf und Spitze einschraubbar sind.

Nach einer bevorzugten Ausführungsform erstreckt sich das Innengewinde über die gesamte Länge des Gewindeabschnittes. Das hat den Vorteil, daß das Rohr auf jede beliebige Länge schneidbar ist, so daß Schrauben gewünschter Länge herstellbar sind, so daß die Lagerhaltung wesentlich verringert werden kann.

Nach einer alternativen Ausführungsform sind die Innenwandung des Gewindeabschnittes 1 und die jeweiligen Schäfte 7 und 11 ohne die jeweiligen Gewinde ausgebildet und in ihren Abmessungen so bestimmt, daß Spitze und Kopf im Paßsitz mit dem Gewindeabschnitt 1 fest verbunden sind.

Alternativ kann an einem Ende ein Gewinde vorgesehen sein und das dazugehörige Element, also Spitze oder Kopf, ein entsprechendes Gewinde aufweisen und einschraubbar sein, so daß an diesem Ende ein Abschneiden des Rohres auf eine vorbestimmte Länge möglich bleibt, und das andere Element, also Kopf oder Spitze kann durch Paßsitz eingebracht sein.

Die Knochenschraube bildende Teile sind vorzugsweise aus Titan geformt.

Die Möglichkeit des Einschraubens von Spitze und Kopf schafft auch den Vorteil, daß in den Hohlraum der so gebildeten Schraube Knochenmaterial oder ein anderes wachstumsförderndes Material eingefüllt werden kann, so daß der Heilungsprozeß sehr beschleunigt wird.

## Patentansprüche

1. Knochenschraube mit einem rohrförmig ausgebildeten Gewindeabschnitt (1), einer Spitze (6, 12) an einem ersten Ende und einem Kopf (8) zum Eingreifen mit einem Schraubendreher am gegenüberliegenden zweiten Ende, wobei die Wandung des Gewindeabschnittes (1) eine Mehrzahl von Ausnehmungen (2, 3) aufweist, und der rohrförmige Gewindeabschnitt (1) auf seiner Außenwandung ein Knochengewinde (4) aufweist, **dadurch gekennzeichnet, dass** wenigstens angrenzend an einem Ende einen Innengewindeabschnitt zum Einschrauben von Kopf (8) bzw. Spitze (6, 12) aufweist.

2. Knochenschraube nach Anspruch 1, **dadurch gekennzeichnet, dass** der Innengewindeabschnitt als metrisches Gewinde (5) ausgebildet ist.

3. Knochenschraube nach nach Anspruch 1 oder 2, **dadurch gekennzeichnet, dass** sich das Innengewinde über die gesamte Länge des Gewindeabschnittes (1) erstreckt.

4. Knochenschraube mit einem rohrförmig ausgebildeten Gewindeabschnitt (1), einer Spitze (12) an einem ersten Ende und einem Kopf (8) zum Eingreifen mit einem Schraubendreher am gegenüberliegenden zweiten Ende, wobei die Wandung des Gewindeabschnittes (1) eine Mehrzahl von Ausnehmungen (2, 3) aufweist, und der rohrförmige Gewindeabschnitt (1) auf seiner Außenwandung ein Knochengewinde (4) aufweist, **dadurch gekennzeichnet, dass** der Kopf (8) und/oder die Spitze (6, 12) so bemessen sind, dass sie im Passsitz in die Enden des Gewindeabschnittes (1) einsetzbar sind.

5. Knochenschraube nach einem der Ansprüche 1 bis 4, **dadurch gekennzeichnet, dass** die Spitze (12) als selbstschneidende Spitze ausgebildet ist.

## Claims

1. Bone screw with a tubular threaded portion (1), a tip (6, 12) at a first end and a head (8), for engagement with a screwdriver, as the opposite, second end, the wall of the threaded portion (1) having a plurality of recesses (2, 3) and the tubular threaded portion (1) having a bone thread (4) on its outer wall, **characterized in that**, at least adjacent to one end, [lacuna] has an internally threaded portion into which the head (8) and/or tip (6, 12) can be screwed.

2. Bone screw according to Claim 1, **characterized in that** the internally threaded portion is in the form of a metric thread (5).

3. Bone screw according to Claim 1 or 2, **characterized in that** the internal thread extends over the entire length of the threaded portion (1).

4. Bone screw with a tubular threaded portion (1), a tip (12 at a first end and a head (8), for engagement with a screwdriver, and the opposite, second end, the wall of the threaded portion (1) having a plurality of recesses (2, 3) and the tubular threaded portion (1) having a bone thread (4) on its outer wall, **characterized in that** the head (8) and/or the tip (6, 12) are/is dimensioned such that they can be inserted with a snug fit into the ends of the threaded portion (1).

5. Bone screw according to one of claims 1 to 4, **characterized in that** the tip (12) is designed as a self-tapping tip.

## Revendications

1. Vis pour os ayant une section filetée réalisée en forme tubulaire (1), une pointe (6, 12) à une première extrémité et une tête (8) pour la coopération avec un tournevis à la seconde extrémité opposée, la paroi de la section filetée (1) présentant une pluralité de creux (2, 3) et la section filetée en forme tubulaire (1) présentant sur sa paroi extérieure un filetage pour l'os (4), **caractérisée en ce qu'**elle présente, au moins au voisinage d'une extrémité, une section taraudée pour le vissage d'une tête (8) ou d'une pointe (6, 12).

2. Vis pour os selon la revendication 1, **caractérisée en ce que** la section taraudée est réalisée sous la forme d'un taraudage au pas métrique (5).

3. Vis pour os selon la revendication 1 ou 2, **caractérisé en ce que** le taraudage s'étend sur toute la longueur de la section filetée (1).

4. Vis pour os ayant une section filetée réalisée en forme tubulaire (1), une pointe (12) à une première extrémité et une tête (8) pour la coopération avec un tournevis à la seconde extrémité opposée, la paroi de la section filetée (1) présentant une pluralité de creux (2, 3) et la section filetée tubulaire (1) présentant sur sa paroi extérieure un filetage pour os (4), **caractérisée en ce que** la tête (8) et/ou la pointe (6, 12) ont des dimensions telles qu'elles peuvent être introduites avec un ajustement fin dans les extrémités de la section filetée (1).

5. Vis pour os selon l'une des revendications 1 à 4, **caractérisée en ce que** la pointe (12) est réalisée sous la forme d'une pointe autotaraudeuse.
